# EUROPEAN PATENT APPLICATION

(11) **EP 3 660 164 A1**
(43) Date of publication of application: **03.06.2020**
(21) Application number: 18209559.6
(22) Date of filing: 30.11.2018
(51) Int. Cl.: C12Q 1/68, C12Q 1/6858

(54) **SCREENING OF GERMPLASM FOR DESIRED POLYMORPHISMS BY ASSAYS TESTED ON SYNTHETIC DNA**

(71) Applicant: KWS SAAT SE & Co. KGaA, 37574 Einbeck (DE)
(72) Inventor: Czarnecki, Olaf, 13086 Berlin (DE); Habekost, Sandra, 37586 Dassel (DE); Kloiber-Maitz, Monika, 37574 Einbeck (DE); Wieckhorst, Silke, 37574 Einbeck (DE)

(57) **Abstract**

The present invention relates to methods for identifying desired allelic variations in a population, for selecting individuals comprising a desired allelic variation, as well as methods for generating individuals comprising a desired allelic variation. These methods include the use synthetic oligonucleotides to optimize or validate detection assays.

## Description

### FIELD OF THE INVENTION

The invention relates to methods for generating, identifying, and/or selecting plants having a particular genotype, such as a particular desired polymorphism.

### BACKGROUND OF THE INVENTION

Plant breeding requires variation in allele composition of germplasm. Variation can be of natural origin based on natural mutation or can be induced by physical or chemical mutation of germplasm. It can also be generated by molecular biology techniques such as transgenic or gene editing approaches. In contrast to the latter molecular biotechniques, natural and induced mutations occur randomly, mostly heterozygous and it cannot be predicted which individual carries a desired polymorphism or mutation.

Generally, DNA polymorphisms can be detected by a set of different molecular techniques, e.g. PCR-based, hybridization-based or sequencing. Except for sequencing these techniques require prior knowledge of the polymorphism to develop polymorphism specific detection assays. Sequencing, however, targeted or next generation sequencing (NGS) are both, labour intensive, costly and not compatible with high-throughput approaches. Especially, NGS performed in high-throughput experiments would additionally require large investments in bioinformatics infrastructure.

Growing knowledge of functions of specific genes in plants leads to the requirement to identify varying alleles of such target genes. Desired allele effects can range from amino acid exchanges to gene knock-outs as well as polymorphisms in regulatory elements like promoters and cis- and trans-acting elements. Such polymorphisms and their effects can be designed in-silica. However, subsequent screen of plant germplasm for such desired mutations is hampered by both, high cost and relatively low throughput. For example, to find a specific single DNA base mutation in a crop that has been induced by chemical mutagenesis (e.g. with ethyl methanesulfonate (EMS) or N-ethyl-N-nitrosourea (ENU)), several millions of mutagenized plants must be screened to find the desired germplasm. If screening cannot be done phenotypically, e.g. because there is no phenotypic assay available or even more likely, there is no visible phenotype, screens must be performed on DNA level. Sequencing millions of individuals is not an option in most cases, due to the high cost and low throughput.

Techniques that are cheaper and can be used in high-throughput suffer the problem that suitable detection assays cannot be tested if the germplasm carrying the desired specific mutation is not present or existing, respectively. Using untested assays to screen millions of plant genomes is unfeasible.

Accordingly, there is a pressing need to provide improved methods for identifying particular mutations, in particular in high throughput settings. The present invention has the objective to solve this problem.

### SUMMARY OF THE INVENTION

The presented invention combines use of relatively cheap and established methods to detect DNA polymorphisms, e.g. KASP markers and microarray-based DNA markers, and previous development of necessary assays on synthetic DNA fragments. It is not limited to DNA marker systems mentioned above but to all past and future methods that detect allelic variation within one DNA sample and where the detection system relies on known DNA polymorphisms. It avoids the use of genotyping systems, e.g. genotyping by sequencing, where the genotyping assays create de novo knowledge about variation.

Synthetic DNA fragments used to develop such marker assays can be two complementary oligonucleotides designed in silico, preferably of up to 120 bp, comprising the DNA polymorphism or mutation and left and right flanking sequences long enough to serve as a template DNA molecule for the respective marker assay. The oligonucleotides cover the region of the fragment amplified by the used marker assay and allow the assay to be validated on actual DNA carrying the desired polymorphism.

This invention can be used whenever DNA marker assays for DNA polymorphisms have to be developed without having the actual DNA polymorphisms in stock or present. This is of particular interest, if large numbers of genotypes need to be screened for specific DNA polymorphisms, e.g. mutant populations and there is a desire to avoid labour intensive and expensive partial or whole genome sequencing.

In an aspect, the invention relates to a method, such as a method for identifying a (desired) allelic variation in a population or a method for selecting an individual comprising a (desired) allelic variation, comprising providing one or more synthetic oligonucleotide comprising a (desired) allelic variation; providing an assay suitable for detecting said (desired) allelic variation; optimizing or validating said assay to ensure that the assay detects said (desired) allelic variation or discriminates between said (desired) allelic variation and a different allelic variation with said synthetic oligonucleotide(s) as a template; and screening the population for the presence of said (desired) allelic variation with said optimized or validated detection assay. Optionally, the method further comprises selecting an individual or individuum comprising said (desired) allelic variation.

In an aspect, the invention relates to a method for generating an individual comprising a (desired) allelic variation in a population, comprising: providing a population; mutagenizing said population; providing one or more synthetic oligonucleotide comprising said (desired) allelic variation; providing an assay suitable for detecting said (desired) allelic variation; optimizing or validating said assay to ensure that the assay detects said (desired) allelic variation or discriminates between said (desired) allelic variation and a different allelic variation with said synthetic oligonucleotide(s) as a template; screening said mutagenized population for the presence of said (desired) allelic variation with said optimized or validated detection assay; selecting an individual comprising said (desired) allelic variation; optionally propagating said individual.

In an aspect, the invention relates to a synthetic oligonucleotide suitable for use in the methods described above.

In an aspect, the invention relates to detection assay suitable for use in the methods described above.

In an aspect, the invention relates to an allele specific primer or probe suitable for use in the methods described above.

In an aspect, the invention relates to use of a synthetic oligonucleotide comprising a (desired) allelic variation for optimizing or validating an assay for detecting said allelic variation in a population.

In an aspect, the invention relates to an individual selected or generated by the methods described above.

### BRIEF DESCRIPTION OF THE FIGURES

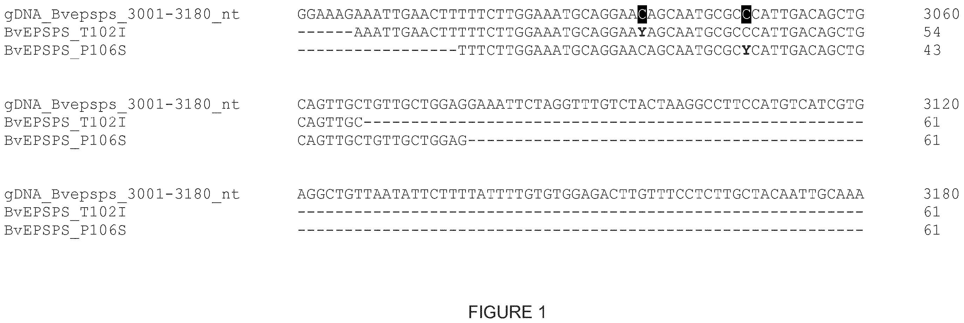

**Figure 1****:** CLUSTAL O (1.2.4) multiple sequence alignment showing the annealing sites of the KASP markers (SEQ ID NOs: 17 and 18) for detection of mutant alleles. Alignment shows only a part of the BvEPSPS gene sequence (SEQ ID NO: 1) from nucleotides 3001 to 3180. Positions where C→T exchanges are desired, are marked with white letter on black background. KASP markers are designed in order to be able to detect C or T (indicated by Y, which can be C or T).

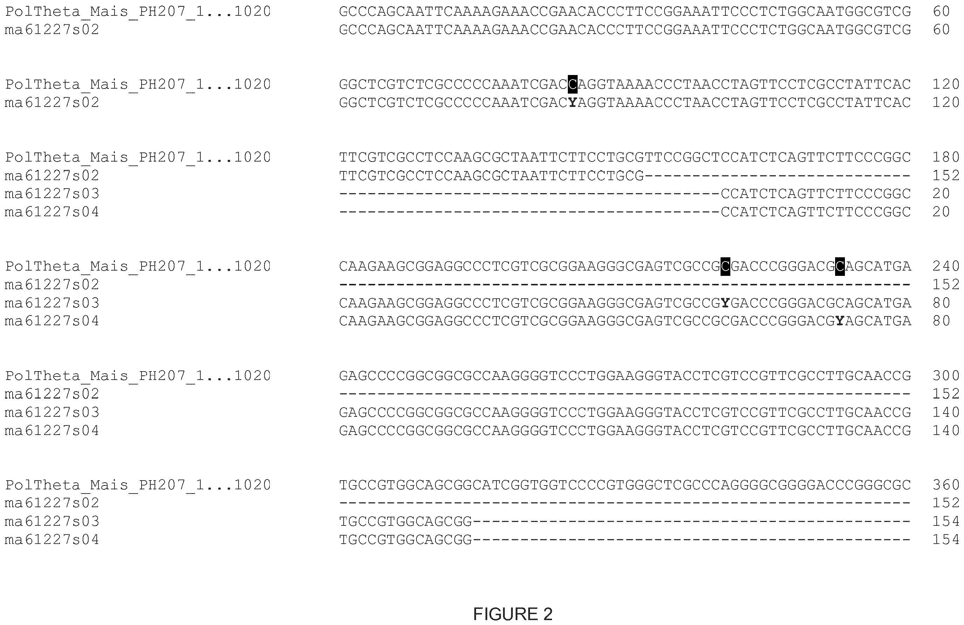

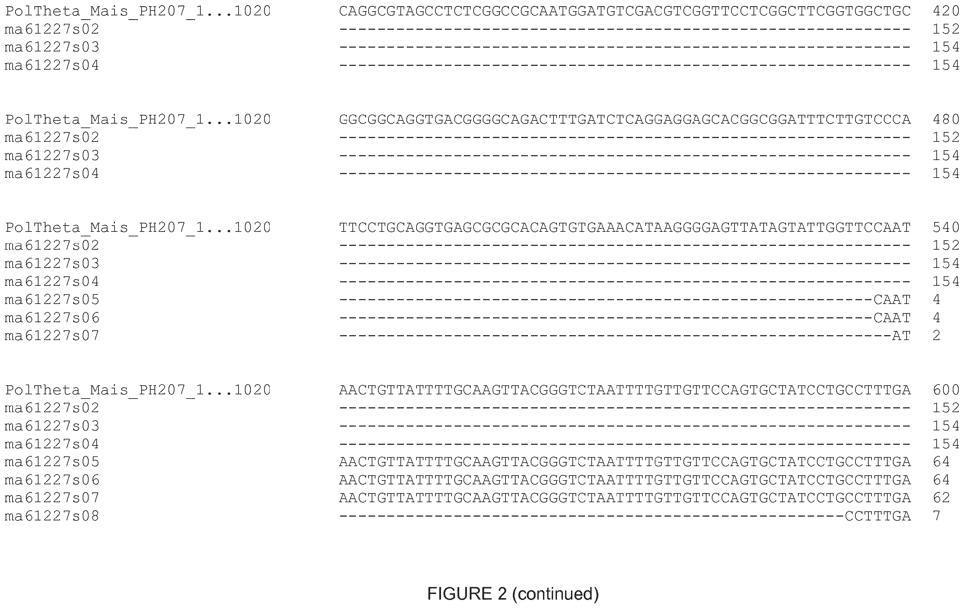

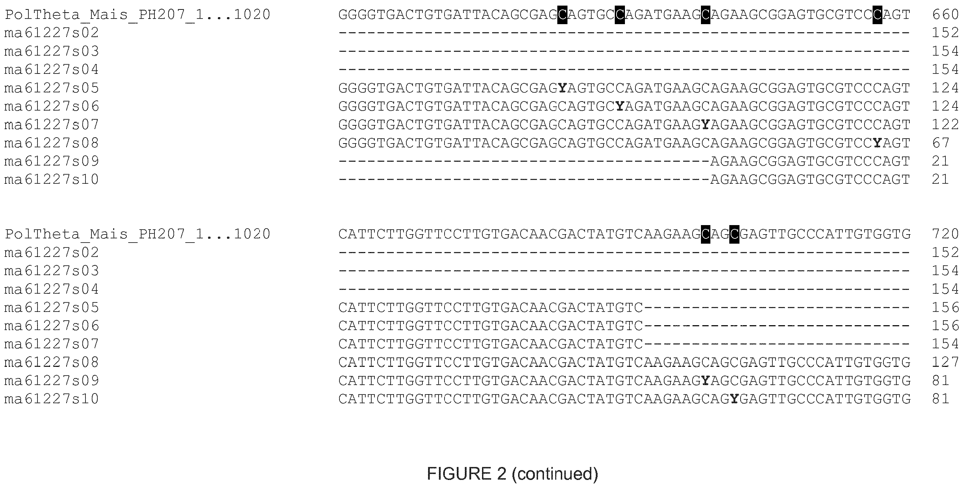

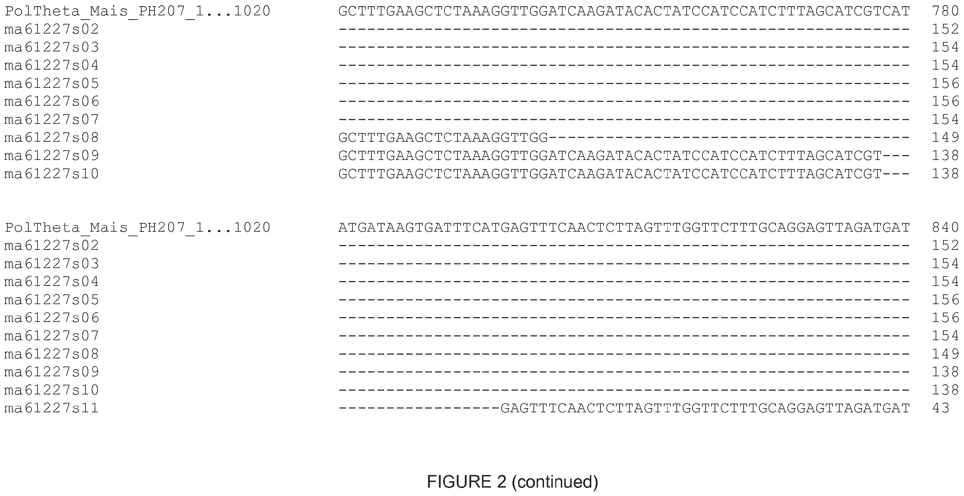

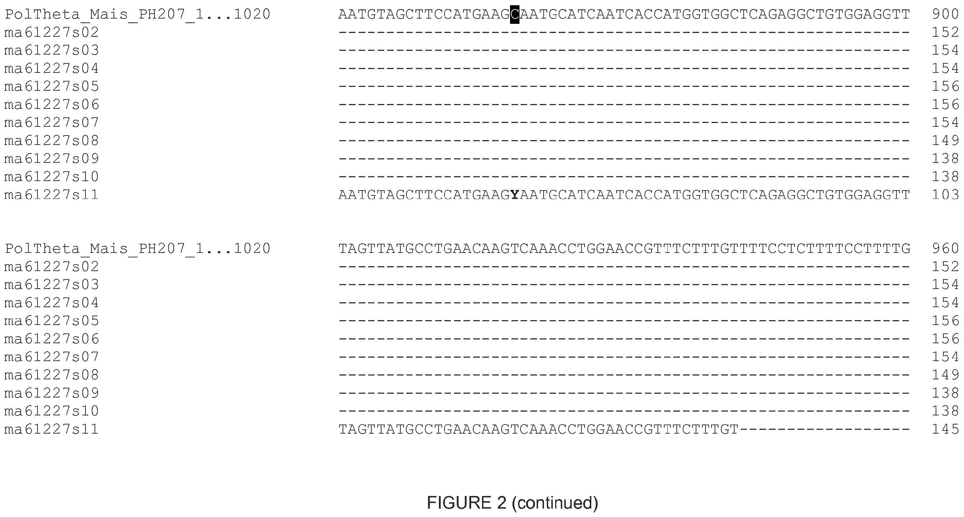

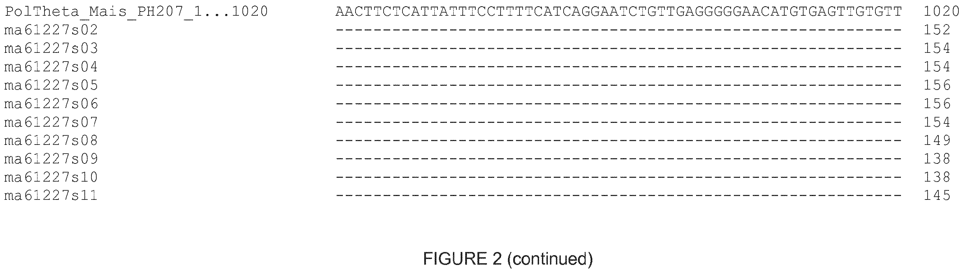

**Figure 2****:** CLUSTAL O (1.2.4) multiple sequence alignment showing the annealing sites of the KASP markers (SEQ ID NOs: 23, 25, 27, 29, 31, 33, 33, 35, 37, 39, 41) for detection of mutant alleles. Alignment shows only a part of the ZmPolTheta gene sequence (SEQ ID NO: 6) from nucleotides 1 to 1020. Positions where C→T exchanges are desired, are marked with white letter on black background. KASP markers are designed in order to be able to detect C or T (indicated by Y, which can be C or T).

### DETAILED DESCRIPTION OF THE INVENTION

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of", as well as the terms "consisting essentially of", "consists essentially" and "consists essentially of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-20% or less, preferably +/-10% or less, more preferably +/-5% or less, and still more preferably +/-1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

Standard reference works setting forth the general principles of recombinant DNA technology include Molecular Cloning: A Laboratory Manual, 2nd ed., vol. 1-3, ed. Sambrook et al., Cold Spring Harbor Laboratory Press, Cold Spring Harbor, N.Y., 1989; Current Protocols in Molecular Biology, ed. Ausubel et al., Greene Publishing and Wiley-Interscience, New York, 1992 (with periodic updates) ("Ausubel et al. 1992"); the series Methods in Enzymology (Academic Press, Inc.); Innis et al., PCR Protocols: A Guide to Methods and Applications, Academic Press: San Diego, 1990; PCR 2: A Practical Approach (M.J. MacPherson, B.D. Hames and G.R. Taylor eds. (1995); Harlow and Lane, eds. (1988) Antibodies, a Laboratory Manual; and Animal Cell Culture (R.I. Freshney, ed. (1987). General principles of microbiology are set forth, for example, in Davis, B. D. et al., Microbiology, 3rd edition, Harper & Row, publishers, Philadelphia, Pa. (1980).

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the following detailed description of the invention, reference is made to the accompanying drawings that form a part hereof, and in which are shown by way of illustration only of specific embodiments in which the invention may be practiced. It is to be understood that other embodiments may be utilised and structural or logical changes may be made without departing from the scope of the present invention. The following detailed description, therefore, is not to be taken in a limiting sense, and the scope of the present invention is defined by the appended claims.

Preferred statements (features) and embodiments of this invention are set herein below. Each statements and embodiments of the invention so defined may be combined with any other statement and/or embodiments unless clearly indicated to the contrary. In particular, any feature indicated as being preferred or advantageous may be combined with any other feature or features or statements indicated as being preferred or advantageous. Hereto, the present invention is in particular captured by any one or any combination of one or more of the below numbered aspects and statements 1 to 52, with any other statement and/or embodiments.
1 - Method for identifying a (desired) allelic variation in a population or for selecting an individuum comprising a desired allelic variation, comprising: providing one or more synthetic oligonucleotide comprising a (desired) allelic variation; providing an assay suitable for detecting said (desired) allelic variation; optimizing and/or validating said assay to ensure that the assay detects said (desired) allelic variation or discriminates between said (desired) allelic variation and a different allelic variation with said synthetic oligonucleotide(s) as a template; screening a population for the presence of said (desired) allelic variation with said optimized or validated detection assay.
2 - Method (according to statement 1) for identifying a (desired) allelic variation in a population, comprising: providing one or more synthetic oligonucleotide comprising said (desired) allelic variation; providing an assay suitable for detecting said (desired) allelic variation; optimizing and/or validating said assay to ensure that the assay detects said (desired) allelic variation or discriminates between said (desired) allelic variation and a different allelic variation with said synthetic oligonucleotide(s) as a template; screening said population for the presence of said (desired) allelic variation with said optimized or validated detection assay.
3 - Method (according to statement 1) for selecting an individuum comprising a (desired) allelic variation in a population, comprising: providing one or more synthetic oligonucleotide comprising said (desired) allelic variation; providing an assay suitable for detecting said (desired) allelic variation; optimizing and/or validating said assay to ensure that the assay detects said (desired) allelic variation or discriminates between said (desired) allelic variation and a different allelic variation with said synthetic oligonucleotide(s) as a template; screening said population for the presence of said (desired) allelic variation with said optimized or validated detection assay; selecting an individuum comprising said (desired) allelic variation.
4 - Method (according to statement 1) for generating an individuum comprising a (desired) allelic variation in a population, comprising: providing a population; mutagenizing said population; providing one or more synthetic oligonucleotide comprising said (desired) allelic variation; providing an assay suitable for detecting said (desired) allelic variation; optimizing and/or validating said assay to ensure that the assay detects said (desired) allelic variation or discriminates between said (desired) allelic variation and a different allelic variation with said synthetic oligonucleotide(s) as a template; screening said mutagenized population for the presence of said (desired) allelic variation with said optimized or validated detection assay; selecting an individuum comprising said (desired) allelic variation; optionally propagating said individuum.
5 - The method according to statement 4, comprising propagating said individuum so as to obtain said (desired) allelic variation homozygous.
6 - The method according to any of statements 1 to 5, wherein said assay is a hybridization-based assay or a PCR-based assay.
7 - The method according to any of statements 1 to 6, wherein said assay is KASP.
8 - The method according to any of statements 1 to 6, wherein said assay is a microarray.
9 - The method according to any of statements 1 to 8, wherein said assay does not comprises sequencing.
10 - The method according to any of statements 1 to 9, wherein optimizing said assay comprises comparing multiple primers or probes.
11 - The method according to any of statements 1 to 10, wherein the optimized assay comprises primers or probes which best discriminate between said (desired) allelic variation and said other allelic variation.
12 - The method according to any of statements 1 to 11, wherein said synthetic oligonucleotide has a length of between 50 and 500 nucleotides, preferably between 80 and 200 nucleotides.
13 - The method according to any of statements 1 to 12, wherein said synthetic oligonucleotide comprises said mutation and 5' and 3' flanking wild type sequences.
14 - The method according to any of statements 1 to 13, wherein said (desired) allelic variation is not naturally present in said population.
15 - The method according to any of statements 1 to 14, wherein said synthetic oligonucleotide is double-stranded.
16 - The method according to any of statements 1 to 15, wherein said synthetic oligonucleotide comprising said (desired) allelic variation is combined with one or more additional synthetic oligonucleotides comprising a different allelic variation.
17 - The method according to statement 12, wherein said different allelic variation is a wild type allele, endogenous allele, native allele, and/or naturally occurring allele.
18 - The method according to any of statements 16 to 17, wherein said different allelic variation is naturally present in said population.
19 - The method according to any of statements 1 to 18, wherein said synthetic oligonucleotide is spiked in genomic DNA.
20 - The method according to statement 19, wherein said genomic DNA is wild type genomic DNA.
21 - The method according to any of statements 19 to 20, wherein said genomic DNA originates from the same species as the population.
22 - The method according to any of statements 1 to 21, wherein said population is mutagenized.
23 - The method according to statement 22, wherein said mutagenized population is obtained by random mutagenesis.
24 - The method according to any of statements 22 to 23, wherein said mutagenesis is ENU or EMS based mutagenesis.
25 - The method according to any of statements 1 to 24, wherein said allelic variation is a mutation.
26 - The method according to statement 25, wherein said mutation is a point mutation.
27 - The method according to any of statements 25 to 26, wherein said mutation is a nonsense mutation.
28 - The method according to any of statements 25 to 27, wherein said mutation is a knockout mutation.
29 - The method according to any of statements 1 to 28, wherein said allelic variation is a SNP.
30 - The method according to any of statements 1 to 29, wherein said population is a population of plants or plant parts.
31 - The method according to statement 30, wherein said plant part is a plant organ or plant cell.
32 - The method according to any of statements 30 to 31, wherein said plant part is a seed.
33 - The method according to any of statements 1 to 32, wherein: said population is a population of sugar beets; said synthetic oligonucleotide comprises the sequence of SEQ ID NO: 19 or 20, or a fragment thereof comprising the sequence of SEQ ID NO: 51 or SEQ ID NO: 52.
34 - The method according to any of statements 1 to 32, wherein: said population is a population of maize; said synthetic oligonucleotide comprises the sequence of SEQ ID NO: 42 or 43, or a fragment thereof comprising the sequence of SEQ ID NO: 53 or SEQ ID NO: 54.
35 - A synthetic oligonucleotide suitable for use in the method according to any of statements 1 to 34.
36 - The synthetic oligonucleotide according to statement 35, wherein said oligonucleotide comprises the sequence of SEQ ID NO: 19 or 20, or a fragment thereof comprising the sequence of SEQ ID NO: 51 or SEQ ID NO: 52.
37 - The synthetic oligonucleotide according to statement 35, wherein said oligonucleotide comprises the sequence of SEQ ID NO: 42 or 43, or a fragment thereof comprising the sequence of SEQ ID NO: 53 or SEQ ID NO: 54.
38 - A detection assay suitable for use in the method according to any of statements 1 to 34.
39 - The detection assay according to statement 38, comprising one or more primers or probes as defined in statement 11.
40 - An allele specific primer or probe suitable for use in the method according to any of statements 1 to 34.
41 - The allele specific primer according to statement 36, which is a KASP primer.
42 - Use of a synthetic oligonucleotide comprising a (desired) allelic variation for optimizing or validating an assay for detecting said allelic variation in a population.
43 - An individuum selected or generated by the method according to any of statements 3 to 34, preferably wherein said individuum is a plant.
44 - A part, seed or progeny of the plant of statement 43, wherein the part, seed or progeny exhibit the (desired) allelic variation.
45 - A maize plant or plant part having a mutation in the polymerase theta gene.
46 - The maize plant or plant part according to statement 45, wherein said mutation is a nonsense mutation.
47 - The maize plant or plant part according to any of statements 45 to 46 wherein said mutation is homozygous or heterozygous.
48 - The maize plant or plant part according to any of statements 45 to 47, comprising any one or more of SEQ ID NOs: 23, 25, 27, 29, 31, 33, 35, 37, 39, or 41, preferably wherein Y is T.
49 - A sugar beet plant or plant part having a mutation in the epsps gene.
50 - The sugar beet plant or plant part according to statement 49, wherein said mutation is a SNP.
51 - The sugar beet plant or plant part according to any of statements 49 to 50 wherein said mutation is homozygous or heterozygous.
52 - The sugar beet plant or plant part according to any of statements 49 to 51, comprising any one or more of SEQ ID NOs: 17 or 18, preferably wherein Y is T.

In an aspect, the invention relates to a method, such as a method for identifying a (desired) allelic variation in a population or a method for selecting an individual comprising a (desired) allelic variation, comprising providing one or more synthetic oligonucleotide comprising a (desired) allelic variation; providing an assay suitable for detecting said (desired) allelic variation; optimizing or validating said assay to ensure that the assay detects said (desired) allelic variation or discriminates between said (desired) allelic variation and a different allelic variation with said synthetic oligonucleotide(s) as a template; and screening a population for the presence of said (desired) allelic variation with said optimized or validated detection assay, and optionally selecting an individual comprising said (desired) allelic variation.

In an aspect, the invention relates to a method, such as a method for identifying a (desired) allelic variation in a population or for selecting an individual comprising a (desired) allelic variation, comprising screening a population for the presence of a (desired) allelic variation with an optimized or validated detection assay, and optionally selecting an individual comprising said (desired) allelic variation; said optimized or validated detection assay obtained by: providing one or more synthetic oligonucleotide comprising said desired allelic variation; providing an assay suitable for detecting said desired allelic variation; and optimizing or validating said assay in order to detects said (desired) allelic variation or discriminate between said desired allelic variation and a different allelic variation with said synthetic oligonucleotide(s) as a template.

In an aspect, the invention relates to a method for generating an individual comprising a (desired) allelic variation in a population, comprising: providing a population; mutagenizing said population; providing one or more synthetic oligonucleotide comprising said (desired) allelic variation; providing an assay suitable for detecting said (desired) allelic variation; optimizing or validating said assay to ensure that the assay detects said (desired) allelic variation or discriminates between said (desired) allelic variation and a different allelic variation with said synthetic oligonucleotide(s) as a template; screening said mutagenized population for the presence of said (desired) allelic variation with said optimized or validated detection assay; selecting an individual comprising said (desired) allelic variation; optionally propagating said individual. In certain embodiments, an individual comprising a (desired) allelic variation can be propagated so as to obtain a homozygous allelic variation. Homozygous allelic variation can be obtained by means well known in the art such as (back)crossing and selecting.

As used herein, the term "population" refers to a group of individuals (or parts thereof) of a particular biological species, subspecies, or variety. A population comprises at least two individuals, preferably at least 10 individuals, more preferably at least 100 individuals, such as at least 1000 individuals or at least 10000 individuals. It will be understood that when referring to a population or individual, also parts or derivatives thereof may be included. The terms "individual" and "individuum" are used herein interchangeably.

In certain embodiments, the part of an individual is an organ, a tissue, a cell or a seed of the individual.

In certain embodiments, the population or individual (or part thereof) is a plant (population). As used herein unless clearly indicated otherwise, the term "plant" intended to mean a plant at any developmental stage.

The term "plant" according to the present invention includes whole plants or parts of such a whole plant. Whole plants preferably are seed plants, or a crop. "Parts of a plant" are e.g. shoot vegetative organs/structures, e.g., leaves, stems and tubers; roots, flowers and floral organs/structures, e.g. bracts, sepals, petals, stamens, carpels, anthers and ovules; seed, including embryo, endosperm, and seed coat; fruit and the mature ovary; plant tissue, e.g. vascular tissue, ground tissue, and the like; and cells, e.g. guard cells, egg cells, pollen, trichomes and the like; and progeny of the same. Parts of plants may be attached to or separate from a whole intact plant. Such parts of a plant include, but are not limited to, organs, tissues, and cells of a plant, and preferably seeds. A "plant cell" is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant. "Plant cell culture" means cultures of plant units such as, for example, protoplasts, cell culture cells, cells in plant tissues, pollen, pollen tubes, ovules, embryo sacs, zygotes and embryos at various stages of development. "Plant material" refers to leaves, stems, roots, flowers or flower parts, fruits, pollen, egg cells, zygotes, seeds, cuttings, cell or tissue cultures, or any other part or product of a plant. This also includes callus or callus tissue as well as extracts (such as extracts from taproots) or samples. A "plant organ" is a distinct and visibly structured and differentiated part of a plant such as a root, stem, leaf, flower bud, or embryo. "Plant tissue" as used herein means a group of plant cells organized into a structural and functional unit. Any tissue of a plant in planta or in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue. In certain embodiments, the part of the plant is selected from the group consisting of leaves, stems, roots, emerged radicles, flowers, flower parts, petals, fruits, pollen, pollen tubes, anther filaments, ovules, embryo sacs, egg cells, ovaries, zygotes, embryos, zygotic embryos, somatic embryos, apical meristems, vascular bundles, pericycles, seeds, roots, and cuttings.

In certain embodiments, the plant is not a plant variety.

In certain embodiments, the population or individual (or part thereof) is selected from or originates from, a plant species selected from the group consisting of: Hordeum vulgare, Hordeum bulbusom, Sorghum bicolor, Saccharum officinarium, Zea mays, Setaria italica, Oryza minuta, Oryza sativa, Oryza australiensis, Oryza alta, Triticum aestivum, Secale cereale, Malus domestica, Brachypodium distach-yon, Hordeum marinum, Aegilops tauschii, Daucus glochidiatus, Beta vulgaris, Daucus pusillus, Daucus muricatus, Daucus carota, Eucalyptus grandis, Nicotiana sylvestris, Nicotiana tomentosiformis, Nicotiana tabacum, Solanum lycopersicum, Solanum tuberosum, Coffea canephora, Vitis vinifera, Erythrante guttata, Genlisea aurea, Cucumis sativus, Morus notabilis, Arabidopsis arenosa, Arabidopsis lyrata, Arabidopsis thaliana, Crucihimalaya himalaica, Crucihimalaya wallichii, Cardamine flexuosa, Lepidium virginicum, Capsella bursa pastoris, Olmarabidopsis pumila, Arabis hirsute, Brassica napus, Brassica oleracea, Brassica rapa, Raphanus sativus, Brassica juncea, Brassica nigra, Eruca vesicaria subsp. sativa, Citrus sinensis, Jatropha curcas, Populus trichocarpa, Medicago truncatula, Cicer yama-shitae, Cicer bijugum, Cicer arietinum, Cicer reticulatum, Cicer judaicum, Cajanus cajanifolius, Cajanus scarabaeoides, Phaseolus vulgaris, Glycine max, Astragalus sinicus, Lotus japonicas, Torenia fournieri, Allium cepa, Allium fistulosum, Allium sativum, and Allium tuberosum.

As used herein, "maize" refers to a plant of the species Zea mays, preferably Zea mays ssp mays.

As used herein, "sugar beet" refers to a plant of the species Beta vulgaris, preferably Beta vulgaris ssp vulgaris. For example, numbering among these are Beta vulgaris subsp. vulgaris var. altissima (sugar beet in a narrower sense), Beta vulgaris ssp. vulgaris var. vulgaris (chard), Beta vulgaris ssp. vulgaris var. conditiva (beetroot / red beet), Beta vulgaris ssp. vulgaris var. crassa/alba (fodder beet).

The term "oligonucleotide" when used herein relates to a (short) DNA or RNA molecule. Oligonucleotides are characterized by the sequence of nucleotide residues that make up the entire molecule. The oligonucleotide may comprise or encompass a marker as defined herein elsewhere. In certain embodiments, the oligonucleotide is an artificial or synthetic oligonucleotide. In certain embodiments, the sequence of the oligonucleotide is not naturally occurring. The oligonucleotide in certain embodiments comprises at least one nucleotide which is not identical to a corresponding nucleotide in an otherwise identical oligonucleotide the sequence of which is naturally occurring. In certain embodiments, the sequence of the oligonucleotide is naturally occurring. In certain embodiments, the oligonucleotide comprises or encodes the desired allelic variation. Depending on the type of assay to detect the allelic variation, the allelic variation may occur at different positions within the oligonucleotide. Typically however, the oligonucleotide may comprise the allelic variation and 5' and 3' flanking sequences. In certain embodiments, the invention provides multiple oligonucleotides. In such embodiments, such multiple oligonucleotides each may comprise a different allelic variation, such as naturally and/or non-naturally occurring allelic variations. The length of the oligonucleotide is not of particular importance. The oligonucleotide according to the present invention however, preferably has a length sufficient that a particular allelic variation can be detected. For instance, the length of the oligonucleotide should preferably be sufficient that assay primers are able to bind/anneal. PCR-based assays need always a forward primer and a reverse primer, such that the sum of these primers is the absolute minimum length of the oligonucleotides in such PCR-based assay. There is no maximum length and is in principle infinite, but typically is limited by the capability (and cost) of oligonucleotide synthesis. The length of the oligonucleotide may for instance also be determined by the amount of allelic variations which need to be (simultaneously) analysed. In particular cases it might be reasonable to synthetize a longer oligonucleotide if this oligonucleotide carries several different mutations on which the marker shall be tested in one go. In certain embodiments, the oligonucleotide ranges from 50 to 500 nucleotides in length, such as from 50 to 450, 50 to 400, 50 to 350, 50 to 300, 50 to 250, 50 to 200, 60 to 500, 60 to 450, 60 to 400, 60 to 350, 60 to 300, 60 to 250, 60 to 200, 70 to 500, 70 to 450, 70 to 400, 70 to 350, 70 to 300, 70 to 250, 70 to 200, 80 to 500, 80 to 450, 80 to 400, 80 to 350, 80 to 300, 80 to 250, or 80 to 200. In certain preferred embodiments, the oligonucleotide has a length ranging from 80 to 200 nucleotides. The oligonucleotide as used herein may or may not encode (part) of a polypeptide or protein. Accordingly, the oligonucleotide may or may not comprise (part) of the coding sequence of a polypeptide or protein. In certain embodiments, the oligonucleotide may correspond to (part of) a non-coding part of a gene. In certain embodiments, the oligonucleotide may correspond to (part of) a regulatory sequence of a gene.

In certain embodiments, the invention provides more than one (synthetic) oligonucleotide, such as two or more (synthetic) oligonucleotides or three or more allelic variations. In certain embodiments, such more than one (synthetic) oligonucleotides each comprise different allelic variations, such as a desired allelic variation and another or other allelic variation(s). In certain embodiments, one (synthetic) oligonucleotide comprises more than one (desired or other) allelic variation. Accordingly, in such embodiments, one (synthetic) oligonucleotide can be used to optimize or validate an assay for detecting multiple (desired or other) allelic variations simultaneously (i.e. with one oligonucleotide). The skilled person will understand that in such case, preferably the multiple allelic variations are in close proximity, such as for instance separated by maximum 500 nucleotides, preferably maximum 400 nucleotides, more preferably maximum 300 nucleotides, even more preferably maximum 200 nucleotides or maximum 150 or 100 nucleotides.

The oligonucleotide according to the invention may be a DNA oligonucleotide or an RNA oligonucleotide, preferably a DNA oligonucleotide. The oligonucleotide according to the invention may be a single-stranded oligonucleotide or a double-stranded oligonucleotide, preferably a double-stranded oligonucleotide. It will be understood that a double-stranded oligonucleotide may nevertheless comprise single-stranded regions, such as for instance at the 5' and/or 3' termini. Typically however, the desired allelic variation resided in a double-stranded portion of the oligonucleotide.

In certain embodiments, the (synthetic) oligonucleotide is spiked in polynucleic acids. Accordingly, the oligonucleotide may be mixed with or present in a mixture with polynucleic acids (different than the oligonucleotide). In certain embodiments, provided is a mixture or composition comprising a (synthetic) oligonucleotide as described herein according to the invention and (different) polynucleic acids. Such composition or mixture can be used in the methods of the invention as described herein. It will be understood that the polynucleic acids referred to may have a different sequence and/or length. In certain embodiments, the polynucleic acids comprise DNA. In certain embodiments, the polynucleic acids comprise genomic DNA, such as fragmented or unfragmented genomic DNA. In certain embodiments, the polynucleic acids are derived from or originate from the same species, subspecies, or variety as the population or individual as described herein elsewhere. In certain embodiments, the polynucleic acids (e.g. genomic DNA) are endogenous, wild type or native polynucleic acids, such as endogenous, wild type or native polynucleic acids derived from or originating from the same species, subspecies, or variety as the population or individual.

In certain embodiments, the invention relates to the identification of an allelic variant or allelic variation. Such variant may be a particular or desired allelic variant, i.e. an allelic variant sought after. The desired allelic variation may be an allelic variation of an endogenous gene (coding or non-coding) or a genetic element, such as a regulatory genetic element, including but not limited to promoters, enhancers, terminators, insulators, etc. This desired allelic variation preferably is associated with or confers a specific trait, preferably an agronomically important phenotype. A desired allelic variation may preferably be characterised by a particular nucleotide and/or protein sequence, such as a particular nucleotide and/or protein sequence of a marker.

As used herein, an "allele" refers to alternative forms of various genetic units associated with different forms of a gene or of any kind of identifiable genetic element, which are alternative in inheritance because they are situated at the same locus in homologous chromosomes. In a diploid cell or organism, the two alleles of a given gene (or marker) typically occupy corresponding loci on a pair of homologous chromosomes. Allelic variants or allelic variations correspond to genes or genetic elements having one or more nucleotide difference(s). Preferably according to the invention, the allelic variants, in particular the desired allelic variant, results in expression of a protein having a different sequence and/or results in a different expression level of a protein. In certain embodiments, the allelic variation is situated in the coding sequence of a gene and results in a variation of one or more amino acid(s).

A variation of one amino acid may be the consequence of for instance a point mutation. Alternatively, the variation may result in the generation of a stop codon, and hence result in a truncated protein. In certain embodiments, the allelic variation is situated in the non-coding sequence of a gene, such as UTR or intron sequence and may affect for instance translation, stability and/or splicing. In certain embodiments, the allelic variation is situated in a regulatory sequence of a gene, such as promoters, enhancers, terminators, insulators, etc and may result in altered expression of a protein or RNA, such as increased or decreased expression. In certain embodiments, the allelic variation is a single nucleotide polymorphism (SNP). An SNP is to be understood as a variation in a single nucleotide that occurs at a specific position in the genome, where each variation is present to some appreciable degree within a population. In certain embodiments, the allelic variation is an indel. An indel is to be understood as being an insertion or deletion of bases in the genome of an organism. In certain embodiments, the indel results in a frameshift, preferably a frameshift in the coding region of a gene. In certain embodiments, the indel may create, abrogate, or otherwise affect the function of a gene or genetic element. The allelic variation as used herein may be naturally occurring or non-naturally occurring. A naturally occurring allelic variation is to be understood to be present at a certain frequency in a given population. In contrast, a non-naturally occurring allelic variation is not present in a given population. In a preferred embodiment, the allelic variation is non-naturally occurring or preferably occurring in less than 1%, such as preferably less than 0.1% of a given population. It will be understood that all these types of mutations described above are encompassed by the term allelic variation as used herein.

The endogenous gene described above in certain embodiments is selected from the group consisting of a gene encoding resistance or tolerance to abiotic stress, including drought stress, osmotic stress, heat stress, cold stress, oxidative stress, heavy metal stress, nitrogen deficiency, phosphate deficiency, salt stress or waterlogging, herbicide resistance, including resistance to glyphosate, glufosinate/phosphinotricin, hygromycin, protoporphyrinogen oxidase (PPO) inhibitors, ALS inhibitors, and Dicamba, a gene encoding resistance or tolerance to biotic stress, including a viral resistance gene, a fungal resistance gene, a bacterial resistance gene, an insect resistance gene, or a gene encoding a yield related trait, including lodging resistance, flowering time, shattering resistance, seed colour, endosperm composition, or nutritional content.

The genetic element described above in certain embodiments is a DNA encoding a non-coding RNA like rRNA, tRNA, miRNA, siRNA, piRNA, snRNA, snoRNA, IncRNA, antisense-RNA, riboswitches or ribozyme, or a regulatory sequence or at least part of a regulatory sequence, wherein the regulatory sequence or the part thereof comprises at least one of a core promoter sequence, a proximal promoter sequence, a cis regulatory sequence, a trans regulatory sequence, a locus control sequence, an insulator sequence, a silencer sequence, an enhancer sequence, a terminator sequence, and/or any combination thereof.

An agronomically important phenotype in the context of the present invention is a phenotype of a plant, which exhibits one or more novel or optimized trait(s) that provide an improved agricultural performance with respect to e.g. yield, biomass, architecture, morphology, fertility, pollen shedding, nutrient partitioning, photosynthesis, carbon sequestration, disease resistance, abiotic and biotic stress tolerance, herbicide tolerance, hormone signalling, and other trait categories. An agronomically important phenotype may be caused by any one allelic variation or a combination of one or more allelic variations in one or more coding, non-coding or regulatory regions of the genetic material of the plant. The modifications may be associated in terms of spatial proximity or genomic context or they may be completely unrelated. An agronomically important phenotype may thus exhibit one or more polygenic traits. The term "sequence" when used herein relates to nucleotide sequence(s), polynucleotide(s), nucleic acid sequence(s), nucleic acid(s), nucleic acid molecule, peptides, polypeptides and proteins, depending on the context in which the term "sequence" is used. The terms "nucleotide sequence(s)", "polynucleotide(s)", "nucleic acid sequence(s)", "nucleic acid(s)", "nucleic acid molecule" are used interchangeably herein and refer to nucleotides, either ribonucleotides or deoxyribonucleotides or a combination of both, in a polymeric unbranched form of any length. Nucleic acid sequences include DNA, cDNA, genomic DNA, RNA, synthetic forms and mixed polymers, both sense and antisense strands, or may contain non-natural or derivatized nucleotide bases, as will be readily appreciated by those skilled in the art.

An "isolated nucleic acid" is understood to be a nucleic acid isolated from its natural or original environment. The term also includes a synthetic manufactured nucleic acid. As used herein, the oligonucleotides according to the invention are preferably isolated nucleic acid sequences.

When used herein, the term "polypeptide" or "protein" (both terms are used interchangeably herein) means a peptide, a protein, or a polypeptide which encompasses amino acid chains of a given length, wherein the amino acid residues are linked by covalent peptide bonds. However, peptidomimetics of such proteins/polypeptides wherein amino acid(s) and/or peptide bond(s) have been replaced by functional analogs are also encompassed by the invention as well as other than the 20 gene-encoded amino acids, such as selenocysteine. Peptides, oligopeptides and proteins may be termed polypeptides. The term polypeptide also refers to, and does not exclude, modifications of the polypeptide, e.g., glycosylation, acetylation, phosphorylation and the like. Such modifications are well described in basic texts and in more detailed monographs, as well as in the research literature.

Amino acid substitutions encompass amino acid alterations in which an amino acid is replaced with a different naturally-occurring amino acid residue. Such substitutions may be classified as "conservative", in which an amino acid residue contained in the wild-type protein is replaced with another naturally-occurring amino acid of similar character, for example Gly↔Ala, Val↔Ile↔Leu, Asp↔Glu, Lys↔Arg, Asn↔Gln or Phe↔Trp↔Tyr. Substitutions encompassed by the present invention may also be "non-conservative", in which an amino acid residue which is present in the wild-type protein is substituted with an amino acid with different properties, such as a naturally-occurring amino acid from a different group (e.g. substituting a charged or hydrophobic amino acid with alanine. "Similar amino acids", as used herein, refers to amino acids that have similar amino acid side chains, i.e. amino acids that have polar, non-polar or practically neutral side chains. "Non-similar amino acids", as used herein, refers to amino acids that have different amino acid side chains, for example an amino acid with a polar side chain is non-similar to an amino acid with a non-polar side chain. Polar side chains usually tend to be present on the surface of a protein where they can interact with the aqueous environment found in cells ("hydrophilic" amino acids). On the other hand, "non-polar" amino acids tend to reside within the center of the protein where they can interact with similar non-polar neighbours ("hydrophobic" amino acids"). Examples of amino acids that have polar side chains are arginine, asparagine, aspartate, cysteine, glutamine, glutamate, histidine, lysine, serine, and threonine (all hydrophilic, except for cysteine which is hydrophobic). Examples of amino acids that have non-polar side chains are alanine, glycine, isoleucine, leucine, methionine, phenylalanine, proline, and tryptophan (all hydrophobic, except for glycine which is neutral).

The term "gene" when used herein refers to a polymeric form of nucleotides of any length, either ribonucleotides or deoxyribonucleotides. The term includes double- and single-stranded DNA and RNA. It also includes known types of modifications, for example, methylation, "caps", substitutions of one or more of the naturally occurring nucleotides with an analog. Preferably, a gene comprises a coding sequence encoding the herein defined polypeptide. A "coding sequence" is a nucleotide sequence which is transcribed into mRNA and/or translated into a polypeptide when placed or being under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleic acid sequences or genomic DNA, while introns may be present as well under certain circumstances.

A used herein, the term "endogenous" refers to a gene or allele which is present in its natural genomic location. The term "endogenous" can be used interchangeably with "native". This does not however exclude the presence of one or more nucleic acid differences with the (reference) wild-type allele. In particular embodiments, the difference with a wild-type allele can be limited to less than 9 preferably less than 6, more particularly less than 3 nucleotide differences. More particularly, the difference with the wildtype sequence can be in only one nucleotide. Preferably, the endogenous allele encodes a modified protein having less than 9, preferably less than 6, more particularly less than 3 and even more preferably only one amino acid difference with the (reference) wild-type protein. Naturally occurring polymorphisms may be considered endogenous, native, and/or wild type. Non-naturally occurring polymorphisms or mutations may be considered exogenous, non-native, or genetically engineered.

As used herein, the term "homozygote" refers to an individual cell or plant having the same alleles (or allelic variation(s)) at one or more or all loci. When the term is used with reference to a specific locus or gene, it means at least that locus or gene has the same alleles (or allelic variation(s)). As used herein, the term "homozygous" means a genetic condition existing when identical alleles (or allelic variation(s)) reside at corresponding loci on homologous chromosomes. As used herein, the term "heterozygote" refers to an individual cell or plant having different alleles (or allelic variation(s)) at one or more or all loci. When the term is used with reference to a specific locus or gene, it means at least that locus or gene has different alleles (or allelic variation(s)). As used herein, the term "heterozygous" means a genetic condition existing when different alleles (or allelic variation(s)) reside at corresponding loci on homologous chromosomes.

A "polymorphism" is a variation in the DNA between two or more individuals within a population. A polymorphism preferably has a frequency of at least 1 % in a population. A useful polymorphism can include a single nucleotide polymorphism (SNP), a simple sequence repeat (SSR), or an insertion/deletion polymorphism, also referred to herein as an "indel". The term "indel" refers to an insertion or deletion, wherein one line may be referred to as having an inserted nucleotide or piece of DNA relative to a second line, or the second line may be referred to as having a deleted nucleotide or piece of DNA relative to the first line.

A "marker" is a (means of finding a position on a) genetic or physical map, or else linkages among markers and trait loci (loci affecting traits). The position that the marker detects may be known via detection of polymorphic alleles and their genetic mapping, or else by hybridization, sequence match or amplification of a sequence that has been physically mapped. A marker can be a DNA marker (detects DNA polymorphisms), a protein (detects variation at an encoded polypeptide), or a simply inherited phenotype (such as the 'waxy' phenotype). A DNA marker can be developed from genomic nucleotide sequence or from expressed nucleotide sequences (e.g., from a spliced RNA or a cDNA). Depending on the DNA marker technology, the marker may consist of complementary primers flanking the locus and/or complementary probes that hybridize to polymorphic alleles at the locus. The term marker locus is the locus (gene, sequence or nucleotide) that the marker detects. "Marker" or "molecular marker" or "marker locus" may also be used to denote a nucleic acid or amino acid sequence that is sufficiently unique to characterize a specific locus on the genome. Any detectable polymorphic trait can be used as a marker so long as it is inherited differentially and exhibits linkage disequilibrium with a phenotypic trait of interest. As used herein, a marker may comprise allelic variations as defined herein elsewhere.

Markers that detect genetic polymorphisms between members of a population are well-established in the art. Markers can be defined by the type of polymorphism that they detect and also the marker technology used to detect the polymorphism. Marker types include but are not limited to, e.g., detection of restriction fragment length polymorphisms (RFLP), detection of isozyme markers, randomly amplified polymorphic DNA (RAPD), amplified fragment length polymorphisms (AFLPs), detection of simple sequence repeats (SSRs), detection of amplified variable sequences of the plant genome, detection of self-sustained sequence replication, or detection of single nucleotide polymorphisms (SNPs). SNPs can be detected e.g. via DNA sequencing, PCR-based sequence specific amplification methods, detection of polynucleotide polymorphisms by allele specific hybridization (ASH), dynamic allele-specific hybridization (DASH), molecular beacons, microarray hybridization, oligonucleotide ligase assays, Flap endonucleases, 5' endonucleases, primer extension, single strand conformation polymorphism (SSCP) or temperature gradient gel electrophoresis (TGGE). DNA sequencing, such as the pyrosequencing technology has the advantage of being able to detect a series of linked SNP alleles that constitute a haplotype. Haplotypes tend to be more informative (detect a higher level of polymorphism) than SNPs.

A "marker allele", alternatively an "allele of a marker locus" or "an allelic variation", can refer to one of a plurality of polymorphic nucleotide sequences found at a marker locus in a population. With regard to a SNP marker, allele refers to the specific nucleotide base present at that SNP locus in that individual plant.

Means for detecting allelic variations are well known in the art. According to the invention, any type of means or assay for detecting allelic variations or nucleotide sequences in general are suitable for practicing the invention. Preferably however, such means do not include DNA or RNA sequencing. Means or assays for detection include for instance without limitation hybridization based methods (such as (dynamic) allele-specific hybridization, molecular beacons, SNP microarrays), enzyme based methods (such as PCR, KASP (Kompetitive Allele Specific PCR), RFLP, ALFP, RAPD, Flap endonuclease, primer extension, 5'-nuclease, oligonucleotide ligation assay), post-amplification methods based on physical properties of DNA (such as single strand conformation polymorphism, temperature gradient gel electrophoresis, denaturing high performance liquid chromatography, high-resolution melting of the entire amplicon, use of DNA mismatch-binding proteins, SNPlex, surveyor nuclease assay), etc. in certain preferred embodiments, a suitable detection assay for practicing the invention is allele-specific PCR, such as including but not limited to KASP (commercialized for instance by LGC; see also He et al. (2014), Methods Mol Biol, 1145:75-86 doi: 10.1007/978-1-4939-0446-4_7; incorporated by reference in its entirety). Allele-specific PCR is well known in the art. By means of further guidance, and without limitation, allele-specific PCR may be performed with three primers: one common reverse primer and two (or more, depending on the number of polymorphisms to be tested) forward primers of which at least one of the primers is chosen from a polymorphic area, with the mutations located at (or near) its 3'-end. Under stringent conditions, a mismatched primer will not initiate replication, whereas a matched primer will. The appearance of an amplification product therefore indicates the genotype. Differential labelling of the forward primers can also distinguish between polymorphisms (or genotypes). By means of further guidance, and without limitation, reference is made to Gaudet et al. (2009), Methods Mol Biol, 578:415-424, doi: 10.1007/978-1-60327-411-1_26, incorporated herein by reference in its entirety. In certain other preferred embodiments, a suitable assay for practicing the invention is allele-specific hybridization, such as with allele-specific oligonucleotides (ASO). Hybridization-based assays may include microarrays.

In an aspect, the invention relates to a detection assay (suitable) for use in the methods of the invention as described herein, such as the methods for identifying a (desired) allelic variation, for selecting an individual comprising a (desired) allelic variation, or for generating an individual comprising a (desired) allelic variation. In certain embodiments, the assay is an allele-specific PCR, such as KASP. In certain embodiments, the assay is an allele-specific hybridization assay. In certain embodiments, the assay is a microarray. Suitable oligonucleotides to be included in the assay are as described herein elsewhere. Suitable primers or probes to be included in the assay are as described herein elsewhere.

As used herein, the term "sequence identity" refers to the degree of identity between any given nucleic acid sequence and a target nucleic acid sequence. Percent sequence identity is calculated by determining the number of matched positions in aligned nucleic acid sequences, dividing the number of matched positions by the total number of aligned nucleotides, and multiplying by 100. A matched position refers to a position in which identical nucleotides occur at the same position in aligned nucleic acid sequences. Percent sequence identity also can be determined for any amino acid sequence. To determine percent sequence identity, a target nucleic acid or amino acid sequence is compared to the identified nucleic acid or amino acid sequence using the BLAST 2 Sequences (BI2seq) program from the stand-alone version of BLASTZ containing BLASTN and BLASTP. This stand-alone version of BLASTZ can be obtained from Fish & Richardson's web site (World Wide Web at fr.com/blast) or the U.S. government's National Center for Biotechnology Information web site (World Wide Web at ncbi.nlm.nih.gov). Instructions explaining how to use the BI2seq program can be found in the readme file accompanying BLASTZ. BI2seq performs a comparison between two sequences using either the BLASTN or BLASTP algorithm.

BLASTN is used to compare nucleic acid sequences, while BLASTP is used to compare amino acid sequences. To compare two nucleic acid sequences, the options are set as follows: -i is set to a file containing the first nucleic acid sequence to be compared (e.g. , C:\seq I .txt); -j is set to a file containing the second nucleic acid sequence to be compared (e.g. , C:\seq2.txt); -p is set to blastn; -o is set to any desired file name (e.g. , C :\output.txt); -q is set to - 1 ; -r is set to 2; and all other options are left at their default setting. The following command will generate an output file containing a comparison between two sequences: C:\B12seq -i c:\seql .txt -j c:\seq2.txt -p blastn -o c:\output.txt -q - 1 -r 2. If the target sequence shares homology with any portion of the identified sequence, then the designated output file will present those regions of homology as aligned sequences. If the target sequence does not share homology with any portion of the identified sequence, then the designated output file will not present aligned sequences. Once aligned, a length is determined by counting the number of consecutive nucleotides from the target sequence presented in alignment with the sequence from the identified sequence starting with any matched position and ending with any other matched position. A matched position is any position where an identical nucleotide is presented in both the target and identified sequences. Gaps presented in the target sequence are not counted since gaps are not nucleotides. Likewise, gaps presented in the identified sequence are not counted since target sequence nucleotides are counted, not nucleotides from the identified sequence. The percent identity over a particular length is determined by counting the number of matched positions over that length and dividing that number by the length followed by multiplying the resulting value by 100. For example, if (i) a 500-base nucleic acid target sequence is compared to a subject nucleic acid sequence, (ii) the BI2seq program presents 200 bases from the target sequence aligned with a region of the subject sequence where the first and last bases of that 200-base region are matches, and (iii) the number of matches over those 200 aligned bases is 180, then the 500-base nucleic acid target sequence contains a length of 200 and a sequence identity over that length of 90% (i.e. , 180 / 200 x 100 = 90). It will be appreciated that different regions within a single nucleic acid target sequence that aligns with an identified sequence can each have their own percent identity. It is noted that the percent identity value is rounded to the nearest tenth. For example, 78.11, 78.12, 78.13, and 78.14 are rounded down to 78.1, while 78.15, 78.16, 78.17, 78.18, and 78.19 are rounded up to 78.2. It also is noted that the length value will always be an integer.

When reference is made to a nucleic acid sequence (e.g. DNA or genomic DNA) having "substantial sequence identity to" a reference sequence or having a sequence identity of at least 80%, e.g. at least 85%, 90%, 95%, 98% or 99% nucleic acid sequence identity to a reference sequence, in one embodiment said nucleotide sequence is considered substantially identical to the given nucleotide sequence and can be identified using stringent hybridisation conditions. In another embodiment, the nucleic acid sequence comprises one or more mutations compared to the given nucleotide sequence but still can be identified using stringent hybridisation conditions.

"Stringent hybridisation conditions" can be used to identify nucleotide sequences, which are substantially identical to a given nucleotide sequence. Stringent conditions are sequence dependent and will be different in different circumstances. Generally, stringent conditions are selected to be about 5°C lower than the thermal melting point (Tm) for the specific sequences at a defined ionic strength and pH. The Tm is the temperature (under defined ionic strength and pH) at which 50% of the target sequence hybridises to a perfectly matched probe. Typically stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridisations (Northern blots using a probe of e.g. 100 nt) are for example those which include at least one wash in 0.2X SSC at 63°C for 20 min, or equivalent conditions. Stringent conditions for DNA-DNA hybridisation (Southern blots using a probe of e.g. 100 nt) are for example those which include at least one wash (usually 2) in 0.2X SSC at a temperature of at least 50°C, usually about 55°C, for 20 min, or equivalent conditions. See also Sambrook et al. (1989) and Sambrook and Russell (2001).

The term "hybridizing" or "hybridization" means a process in which a single-stranded nucleic acid molecule attaches itself to a complementary nucleic acid strand, i.e. agrees with this base pairing. Standard procedures for hybridization are described, for example, in Sambrook et al. (Molecular Cloning. A Laboratory Manual, Cold Spring Harbor Laboratory Press, 3rd edition 2001). Preferably this will be understood to mean an at least 50%, more preferably at least 55%, 60%, 65%, 70%, 75%, 80% or 85%, more preferably 90%, 91 %, 92%, 93%, 94%, 95%, 96%, 97%, 98% or 99% of the bases of the nucleic acid strand form base pairs with the complementary nucleic acid strand. The possibility of such binding depends on the stringency of the hybridization conditions. The term "stringency" refers to hybridization conditions. High stringency is if base pairing is more difficult, low stringency, when a base-pairing is facilitated. The stringency of hybridization conditions depends for example on the salt concentration or ionic strength and temperature. Generally, the stringency can be increased by increasing the temperature and / or decreasing salinity. "Stringent hybridization conditions" are defined as conditions in which hybridization occurs predominantly only between homologous nucleic acid molecules. The term "hybridization conditions" refers not only to the actual binding of the nucleic acids at the prevailing conditions, but also in the subsequent washing steps prevailing conditions. Stringent hybridization conditions are, for example, conditions under which predominantly only those nucleic acid molecules having at least 70%, preferably at least 75%, at least 80%, at least 85%, at least 90% or at least 95% sequence identity hybridize. Less stringent hybridization conditions include: hybridization in 4 x SSC at 37 °C, followed by repeated washing in 1 x SSC at room temperature. Stringent hybridization conditions include: hybridization in 4 x SSC at 65 ° C, followed by repeated washing in 0.1 x SSC at 65 ° C for a total of about 1 hour.

In an aspect, the methods of the invention as described herein comprise optimization and/or validation of a means or assay for detecting allelic variations, such as a desired allelic variation as described herein elsewhere. Such optimization or validation may be performed with the (synthetic) oligonucleotide, as described herein elsewhere, as a template. As used herein, optimization may include increasing the specificity and/or sensitivity of the assay. The terms specificity and sensitivity have their ordinary meaning in the art. By means of further guidance, and without limitation, specificity refers to the true negative rate and measures the proportion of actual negatives that are correctly identified as such. By means of further guidance, and without limitation, sensitivity refers to the true positive rate, the recall, or probability of detection and measures the proportion of actual positives that are correctly identified as such. Similarly, validation may include measurement of specificity and/or sensitivity and optionally comparing such sensitivity/specificity with a predetermined threshold. If the threshold is reached, the assay is validated. Validation and/or optimization may include verification that the assay discriminates or can discriminate between different alleles, such as between a desired allelic variation and another allelic variation. Accordingly, in certain embodiments, validation and/or optimization of the assay comprises performing the assay with a (synthetic) oligonucleotide comprising a particular (desired) allelic variation as a template and comparing the with an assay performed with a (synthetic) oligonucleotide comprising another allelic variation as a template. In certain embodiments, optimization and/or validation of the assay comprises performing the assay with a (synthetic) oligonucleotide comprising a particular (desired) allelic variation as a template and a (synthetic) oligonucleotide comprising another allelic variation as a template. Detection of and/or discrimination between the (desired) allelic variation and another allelic variation validates the assay or indicates an optimized assay. In certain embodiments, in particular in PCR-based or hybridization-based assays, validation and/or optimization comprise comparing different primers (forward and/or reverse) or probes and selecting those primers or probes which discriminate between a particular (desired) allelic variation and another allelic variation, preferably those primers or probes which discriminate best between a particular (desired) allelic variation and another allelic variation. In certain embodiments, in particular in PCR-based or hybridization-based assays, validation and/or optimization comprise selecting primers (forward and/or reverse) or probes and which discriminate between a particular (desired) allelic variation and another allelic variation, preferably those primers or probes which discriminate best between a particular (desired) allelic variation and another allelic variation. In certain embodiments, in particular in PCR-based or hybridization-based assays, validation and/or optimization comprise selecting primers (forward and/or reverse) or probes and which are capable of detecting a particular (desired or other) allelic variation. In all these embodiments, preferably the primers or probes are selected or designed which have increased or optimal selectivity and/or specificity. The skilled person will understand that an "optimal" sensitivity and/or specificity to a certain extent may be tentative. Nevertheless, the skilled person will be capable of selecting or designing primers or probes which are capable of detecting a particular (desired) allelic variation or (optimally) discriminating between a particular (desired) allelic variation and another allelic variation. The skilled person will understand that heuristics may be involved in validating and/or optimizing the assay.

In certain embodiments, the individuals or population(s) as described herein are mutagenized.

In certain embodiments, mutagenesis is random mutagenesis. Cells or organisms may be exposed to mutagens such as UV radiation, X rays or mutagenic chemicals (such as for instance such as ethyl methanesulfonate (EMS)), and mutants with desired characteristics are then selected. Mutants can for instance be identified by TILLING (Targeting Induced Local Lesions in Genomes). The method combines mutagenesis, such as mutagenesis using a chemical mutagen such as ethyl methanesulfonate (EMS) with a sensitive DNA screening-technique that identifies single base mutations/point mutations in a target gene. The TILLING method relies on the formation of DNA heteroduplexes that are formed when multiple alleles are amplified by PCR and are then heated and slowly cooled. A "bubble" forms at the mismatch of the two DNA strands, which is then cleaved by a single stranded nuclease. The products are then separated by size, such as by HPLC. See also McCallum et al.; Nat Biotechnol. 2000 Apr;18(4):455-7 and McCallum et al.; Plant Physiol. 2000 Jun;123(2):439-42; both incorporated herein by reference in their entirety.

In certain embodiments, mutagenesis is site-directed mutagenesis, such as site-directed gene editing. "Gene editing" or "genome editing" refers to genetic engineering in which in which DNA or RNA is inserted, deleted, modified or replaced in the genome of a living organism. Gene editing may comprise targeted or non-targeted (random) mutagenesis. Targeted mutagenesis may be accomplished for instance with designer nucleases, such as for instance with meganucleases, zinc finger nucleases (ZFNs), transcription activator-like effector-based nucleases (TALEN), the clustered regularly interspaced short palindromic repeats system like CRISPR/Cas9, CRISPR/Cpf1, CRISPR/Csm1, CRISPR/MAD7, and programmable base editors for transitions from C•G to T•A base pairs and transitions from A•T to G•C base pairs like CRISPR base editors. These nucleases create site-specific double-strand breaks (DSBs) at desired locations in the genome. The induced double-strand breaks are repaired through nonhomologous end-joining (NHEJ) or homologous recombination (HR), resulting in targeted mutations or nucleic acid modifications. The use of designer nucleases is particularly suitable for generating gene knockouts or knockdowns. Delivery and expression systems of designer nuclease systems as well as programmable base editors are well known in the art.

In certain embodiments, the population or individual as described herein comprises one or more mutation(s) or polymorphism(s). In certain embodiments, each member or individual of the population comprises one or more mutation(s) or polymorphism(s). It is to be understood that such mutation(s) or polymorphism(s) may be considered (an) allelic variation(s) as described herein elsewhere. In certain embodiments, said mutation or polymorphism is a point mutation. In certain embodiments, said mutation or polymorphism is an indel. In certain embodiments, said mutation or polymorphism results in a frameshift of a protein coding sequence. In certain embodiments, said mutation or polymorphism is a nonsense mutation. In certain embodiments, said mutation or polymorphism results in a knockout or knockdown of a gene and/or protein. In certain embodiments, said mutation or polymorphism is a single nucleotide polymorphism (SNP). It will be understood that an individual or population may comprise one or more of the same or different types of mutation as described above.

In an aspect, the invention relates to a (synthetic) oligonucleotide comprising or consisting of the sequence of SEQ ID NO: 19 or 20 or the reverse complement, or a fragment thereof comprising the sequence of SEQ ID NO: 51 and/or SEQ ID NO: 52.

In an aspect, the invention relates to a (synthetic) oligonucleotide comprising or consisting of the sequence of SEQ ID NO: 17 or 18 or the reverse complement, or a fragment thereof of at least 10 nucleotides and comprising Y.

In an aspect, the invention relates to a (synthetic) oligonucleotide comprising or consisting of the sequence of SEQ ID NO: 45 or 46, or a fragment thereof comprising the reverse complement of the sequence of SEQ ID NO: 51 and/or SEQ ID NO: 52.

In an aspect, the invention relates to a (synthetic) oligonucleotide comprising or consisting of the sequence of SEQ ID NO: 44 or the reverse complement, or a fragment thereof comprising at least 12, preferably at least 15, more preferably at least 18 contiguous nucleotides. Preferably said oligonucleotide is a primer.

In an aspect, the invention relates to a (synthetic) oligonucleotide comprising or consisting of a fragment of the sequence of SEQ ID NO: 17 or 18, or the reverse complement, comprising at least 12, preferably at least 15, more preferably at least 18 contiguous nucleotides and comprising Y, preferably as the most 3' or second most 3' nucleotide. Preferably said oligonucleotide is a primer.

In an aspect, the invention relates to a (synthetic) oligonucleotide comprising or consisting of the sequence of SEQ ID NO: 42 or 43 or the reverse complement, or a fragment thereof comprising the sequence of SEQ ID NO: 53 or SEQ ID NO: 54.

In an aspect, the invention relates to a (synthetic) oligonucleotide comprising or consisting of the sequence of SEQ ID NO: 49 or 50, or a fragment thereof comprising the reverse complement of the sequence of SEQ ID NO: 53 or SEQ ID NO: 54.

In an aspect, the invention relates to a (synthetic) oligonucleotide comprising or consisting of the sequence of SEQ ID NO: 23, 25, 27, 29, 31, 33, 35, 37, 39, or 41 or the reverse complement, or a fragment thereof of at least 10 nucleotides and comprising Y.

In an aspect, the invention relates to a (synthetic) oligonucleotide comprising or consisting of a fragment of the sequence of SEQ ID NO: 23 or 25, or the reverse complement, comprising at least 12, preferably at least 15, more preferably at least 18 contiguous nucleotides and comprising Y, preferably as the most 3' or second most 3' nucleotide. Preferably said oligonucleotide is a primer.

In an aspect, the invention relates to a (synthetic) oligonucleotide comprising or consisting of the sequence of SEQ ID NO: 47 or 48, or the reverse complement, or a fragment thereof comprising at least 12, preferably at least 15, more preferably at least 18 contiguous nucleotides. Preferably said oligonucleotide is a primer.

It will be understood that when referring to "Y" in the above oligonucleotides, such is meant to be C or T and refers to the Y position as present in the indicated SEQ ID NOs. The (oligo)nucleotides described above can be used in the methods according to the invention as described herein, such as the methods for identifying a (desired) allelic variation, for selecting an individual comprising a (desired) allelic variation, or for generating an individual comprising a (desired) allelic variation. In certain embodiments, such oligonucleotides have a length as described herein elsewhere. In certain embodiments, the fragments have a length of at least 10 nucleotides, preferably at least 15 nucleotides, more preferably at least 20 nucleotides.

The aspects and embodiments of the invention are further supported by the following non-limiting examples.

### EXAMPLES

**Table 1: description of the sequences used herein**

| **SEQ ID NO** | **Description** |
|---|---|
| SEQ ID NO: 1 | genomic DNA of wild type epsps gene |
| SEQ ID NO: 2 | cDNA of wild type epsps gene |
| SEQ ID NO: 3 | exon 2 of wild type epsps gene |
| SEQ ID NO: 4 | wild type EPSPS protein |
| SEQ ID NO: 5 | genomic DNA of mutant epsps gene (T102I) |
| SEQ ID NO: 6 | cDNA of mutant epsps gene (T102I) |
| SEQ ID NO: 7 | exon 2 of mutant epsps gene (T102I) |
| SEQ ID NO: 8 | mutant EPSPS protein (T102I) |
| SEQ ID NO: 9 | genomic DNA of mutant epsps gene (P106S) |
| SEQ ID NO: 10 | cDNA of mutant epsps gene (P106S) |
| SEQ ID NO: 11 | exon 2 of mutant epsps gene (P106S) |
| SEQ ID NO: 12 | mutant EPSPS protein (P106S) |
| SEQ ID NO: 13 | genomic DNA of mutant epsps gene (T102I and P106S) |
| SEQ ID NO: 14 | cDNA of mutant epsps gene (T102I and P106S) |
| SEQ ID NO: 15 | exon 2 of mutant epsps gene (T102I and P106S) |
| SEQ ID NO: 16 | mutant EPSPS protein (T102I and P106S) |
| SEQ ID NO: 17 | marker BvEPSPS T102I |
| SEQ ID NO: 18 | marker BvEPSPS P106S |
| SEQ ID NO: 19 | synthetic oligo simulating SNP leading to P106S mutation of BvEPSPS |
| SEQ ID NO: 20 | synthetic oligo simulating SNP leading to T102I mutation of BvEPSPS |
| SEQ ID NO: 21 | genomic DNA of wild type pol theta gene |
| SEQ ID NO: 22 | genomic DNA of mutant pol theta gene (C→T at position 85) |
| SEQ ID NO: 23 | ma61227s02 |
| SEQ ID NO: 24 | genomic DNA of mutant pol theta gene (C→T at position 221) |
| SEQ ID NO: 25 | ma61227s03 |
| SEQ ID NO: 26 | genomic DNA of mutant pol theta gene (C→T at position 233) |
| SEQ ID NO: 27 | ma61227s04 |
| SEQ ID NO: 28 | genomic DNA of mutant pol theta gene (C→T at position 624) |
| SEQ ID NO: 29 | ma61227s05 |
| SEQ ID NO: 30 | genomic DNA of mutant pol theta gene (C→T at position 630) |
| SEQ ID NO: 31 | ma61227s06 |
| SEQ ID NO: 32 | genomic DNA of mutant pol theta gene (C→T at position 639) |
| SEQ ID NO: 33 | ma61227s07 |
| SEQ ID NO: 34 | genomic DNA of mutant pol theta gene (C→T at position 657) |
| SEQ ID NO: 35 | ma61227s08 |
| SEQ ID NO: 36 | genomic DNA of mutant pol theta gene (C→T at position 699) |
| SEQ ID NO: 37 | ma61227s09 |
| SEQ ID NO: 38 | genomic DNA of mutant pol theta gene (C→T at position 702) |
| SEQ ID NO: 39 | ma61227s10 |
| SEQ ID NO: 40 | genomic DNA of mutant pol theta gene (C→T at position 859) |
| SEQ ID NO: 41 | ma61227s11 |
| SEQ ID NO: 42 | synthetic oligo simulating C85T mutation of pol theta gene of Zea mays |
| SEQ ID NO: 43 | synthetic oligo simulating C221T mutation of pol theta gene of Zea mays |
| SEQ ID NO: 44 | common primer for marker BvEPSPS T102I or marker BvEPSPS P106S |
| SEQ ID NO: 45 | reverse sequence of SEQ ID NO: 19 |
| SEQ ID NO: 46 | reverse sequence of SEQ ID NO: 20 |
| SEQ ID NO: 47 | common primer for marker ma61227s02 |
| SEQ ID NO: 48 | common primer for marker ma61227s03 |
| SEQ ID NO: 49 | reverse sequence of SEQ ID NO: 42 |
| SEQ ID NO: 50 | reverse sequence of SEQ ID NO: 43 |
| SEQ ID NO: 51 | fragment of SEQ ID NO: 19 |
| SEQ ID NO: 52 | fragment of SEQ ID NO: 20 |
| SEQ ID NO: 53 | fragment of SEQ ID NO: 42 |
| SEQ ID NO: 54 | fragment of SEQ ID NO: 43 |

### EXAMPLE 1: Screening for SNPs leading to amino acid exchanges

Natural occurring tolerance against the herbicide glyphosate is caused by amino acid exchanges in the 5-enolpyruvylshikimate-3-phosphate synthase enzyme. Two DNA polymorphisms have been described based on glyphosate resistant weeds (Yu Q., Jalaludin A., Han H., Chen M., Sammons R. D. & Powles S. B. (2015): Evolution of a double amino acid substitution in the 5-enolpyruvylshikimate-3-phosphate synthase in Eleusine indica conferring high-level glyphosate resistance. Plant Physiol. 167, 1440-1447.). Both the EPSPS proline to serine exchange at amino acid 106 (P106S, position is referenced to EPSPS Arabidopsis thaliana, corresponding to position 179 in EPSPS of Beta vulgaris) and threonine to isoleucine exchange at amino acid 102 (T102I, position is referenced to EPSPS Arabidopsis thaliana, corresponding to position 175 in EPSPS of Beta vulgaris) are based on C to T mutations in the sequence of BvEPSPS (SEQ ID NO: 1).

Sugar beet germplasm was mutagenized using EMS and ENU to induce DNA base pair mutations to generate both mutations in sugar beet (detailed description see below). Statistical calculations, that are not described here, indicated that in total up to 30 million individual M2-mutants need to be screened to find the desired mutations with a 70% chance.

### Detailed description of mutagenesis of sugar beets:

Several kg Seeds of sugar beet elite line T807 (3BT1760, M0 population) were mutagenized with 0.5% EMS and 0.3% and 0.5%, respectively ENU and subsequently drilled for steck production. Stecks were replanted in the following year for seed production. M1 population sizes were 75,000 seed producing plants for EMS and 19,000 plants for ENU mutagenized seeds. We obtained the following M2 seed amounts (values given for purified seed): 240 kg for 0.5% EMS (ident A), 548 kg for 0.3% ENU (ident B), and 256 kg 0.5% ENU (ident C).

176 kg of ident A and 75 kg of idents B and C were sown on a field site. After one month the entire field was sprayed with 0.88 l/ha ROUNDUP MAX (679 g/l active ingredient).

This treatment of 35 million mutants with glyphosate in the field revealed a total amount of approximately 500 candidate plants to be tolerant against glyphosate (=0.0014%). To check whether or not the selected mutants carry either the T102I (SEQ ID NOs: 5-8) or P106S mutation (SEQ ID NOs: 9-13), KASP markers for the respective C/T polymorphisms were developed (Table 2). Sequences for both markers are provided in SEQ ID NOs: 17 and 18 wherein 'Y' in the sequence indicates the SNP to be detected. That means for instances that from SEQ ID NO: 17 one primer carrying a 'C' at the position of 'Y' is able to detect the non-mutated epsps gene and another primer carrying a 'T' is able to detect the mutated epsps gene. In priniciple, it is also possible to use the complementary sequences of SEQ ID NOs: 17 and 18, also called the reverse sequences, which are directed to the other genomic DNA strand. If used as KASP marker additionally a common primer is needed, which can be for example SEQ ID NO: 44 or the complementary sequence thereof. To test the KASP assay prior to application on the mutants, two synthetic DNA oligos were designed and synthetized (SEQ ID NOs: 19 and 20). Since the synthetic DNA oligos are double stranded molecules the corresponding reverse strands are set forth in SEQ ID NO: 45 and 46, respectively. They comprise up to 120 bp of the sugar beet EPSPS (BvEPSPS) genomic sequence carrying either base T or base C at the respective positions. These synthetic oligos served as a template to test the KASP assay quality and capacity to differentiate between both alleles in particular in presence of background sugar beet DNA. After this validation step, these KASP markers have been used to detect the desired mutations on the 500 glyphosate tolerant candidate plants. As it turned out, one plant carried the desired P106S mutation (=0.000003%; 1 out of 35,000,000). Efficiency and quality of the method was tested by Sanger sequencing of exon 2 of the BvEPSPS in all 500 glyphosate tolerant plants (SEQ ID NOs: 3 and 11). Presence of the P106S mutation in the one individual identified by KASP markers was confirmed and absence of this polymorphism in all other plants was confirmed, too.

**Table 2: KASP markers designed for the detection and the differentiation of mutant alleles for glyphosate resistance in Beta vulgaris DNA background. Exemplary, as common primer a sequence as set forth in SEQ ID NO: 44 or a complementary sequence thereof can be used.**

| marker name | SEQ ID NO: | Length of the marker (bp) | Position of C→T mutation in epsps gene of *B. vulgaris* (SEQ ID NO: 1) | Exemplary detectable mutant sequence of epsps (SEQ ID NO) |
|---|---|---|---|---|
| BvEPSPS T102I | 17 | 61 | 3037 | 5; 13 |
| BvEPSPS P106S | 18 | 61 | 3048 | 9; 13 |

As another example, several amino acid substitution mutations in CENH3 are known to cause haploid induction in plants like rapeseed and Arabidopsis (EP 3 186 381, EP 3 237 623). Transfer of this technology to other crops, especially to maize, sunflower or sugar beet is of high interest. As CENH3 is highly conserved within the plant kingdom, it is possible to identify the amino acids of interest in other crops in sequence alignments. Depending on the mutagenic agent used to establish the mutagenized population, different nucleotide exchanges can be expected, which can be translated into amino acid exchanges. The most common are (Silkara P, Chawade A., Larsson M., Olsson J., Olsson O. (2011): Mutagenesis as a tool in plant genetics, functional genomics, and breeding. Int J Plant Genomics. 2011:314829):
- EMS: C→T and G-A
- ENU: C→T, G→A, A→T and T-A
- Az-MNU: C→T, G→A, A→G and T→C

The most frequent nucleotide exchanges in a specific population likely depends on species, treatment and variety and must be determined by sequencing. If a desired DNA polymorphism can be introduced by mutagenesis of a certain mutagen/genotype population, KASP markers on such mutations can be developed as shown above and can significantly improve the screening process for desired mutations in the TILLING population.

### EXAMPLE 2: Screening for SNPs leading to pre-mature stop of translation

Except for very specific mutations as shown for EPSPS and CENH3, the most frequent application of this technology will be the search/screen for knock-out mutations. For example, by EMS mutagenesis, the following five codons can be converted to stop codons: CAA, CAG, TGG, CGA and TGG. If 10 markers for potential stop codons are designed in the first half of the protein of interest, the probability to detect at least one knock out mutant in 10,000 plants in a tilling population of any crop with a mutation frequency of 1 mutation in 150 kb is between 70 and 80% depending on the GC content of the gene. In this example, the method is applied for instances to identify knock-out mutations for the gene of polymerase theta (Pol theta) in corn (Zea mays). Such germplasm with a deficient Pol theta shows a reduction of non-homologous recombination and of stabile integration of T-DNA during a transformation process (WO 2017164738).

For the validation of KASP assays according to the present invention for mutagenic agent-based TILLING approaches the DNA sequence of Pol theta gene (SEQ ID NO: 21) derived from Maize genotype PH207 (as described in "Draft Assembly of Elite Inbred Line PH207 Provides Insights into Genomic and Transcriptome Diversity in Maize", Hirsch et al., Plant Cell. 2016 Nov; 28(11): 2700-2714. Published online 2016 Nov 1. doi: 10.1105/tpc.16.00353) which should include the desired knock-out mutation as SNP, was used to design primers for the KASP analysis with the algorithm included in the KRAKEN software package of LGC Genomics (www.lgcgroup.com/products/genotyping-software/kraken/). Two synthetic oligos in forward (SEQ ID NOs: 42 and 43) and reverse complement direction (SEQ ID NOs: 49 and 50) were subsequently generated covering exemplary the sequence of the design of ma61227s02 and ma61227s03s (Table 3) and carrying the desired nucleotide for the knock-out mutation. In this example the knock-out mutations base on the exchange of a cytosine (C) for a thymine (T) (C→T) which can be made by any of above mentioned mutagenic agents. Both synthetic oligos were mixed in an equimolar ratio and used for KASP-analysis in an end concentration of 0.01 pM as well as spiked in genomic DNA of a wildtype genotype to simulate heterozygous pattern.

**Table 3: KASP markers designed for the detection knock-out mutant alleles for Pol Theta in Zea mays DNA background. Exemplary for ma61227s02, as common primer a sequence as set forth in SEQ ID NO: 47 or a complementary sequence thereof can be used. Exemplary for ma61227s03, as common primer a sequence as set forth in SEQ ID NO: 48 or a complementary sequence thereof can be used.**

| KASP Design | marker name | SEQ ID NO: | Length of the marker | Position of C→T mutation in *Pol theta* gene (referenced to SEQ ID NO: 21) | Exemplary detectable mutant sequence of pol theta (SEQ ID NO) |
|---|---|---|---|---|---|
| 1 | ma61227s02 | 23 | 152 | 85 | 22 |
| 2 | ma61227s03 | 25 | 154 | 221 | 24 |
| | ma61227s04 | 27 | 154 | 233 | 26 |
| 3 | ma61227s05 | 29 | 156 | 624 | 28 |
| | ma61227s06 | 31 | 156 | 630 | 30 |
| | ma61227s07 | 33 | 154 | 639 | 32 |
| | ma61227s08 | 35 | 149 | 657 | 34 |
| | ma61227s09 | 37 | 138 | 699 | 36 |
| | ma61227s10 | 39 | 138 | 702 | 38 |
| 4 | ma61227s11 | 41 | 145 | 859 | 40 |

The KASP-design with the best separation of the clusters with homozygous synthetic template, the heterozygous spiked samples and the cluster with homozygous wildtype allele were then chosen to analyse a produced maize TILLING population.

## Claims

1. Method for identifying a desired allelic variation in a population or for selecting an individuum comprising a desired allelic variation, comprising:
providing a synthetic oligonucleotide comprising said desired allelic variation;
providing an assay suitable for detecting said desired allelic variation;
optimizing or validating said assay to ensure that the assay discriminates between said desired allelic variation and a different allelic variation with said synthetic oligonucleotide as a template;
screening said population for the presence of said desired allelic variation with said optimized or validated detection assay; and
optionally selecting an individual comprising said desired allelic variation.

2. Method for generating an individual comprising a (desired) allelic variation in a population, comprising:
providing a population;
mutagenizing said population;
providing one or more synthetic oligonucleotide comprising said (desired) allelic variation;
providing an assay suitable for detecting said (desired) allelic variation;
optimizing or validating said assay to ensure that the assay detects said (desired) allelic variation or discriminates between said (desired) allelic variation and a different allelic variation with said synthetic oligonucleotide(s) as a template;
screening said mutagenized population for the presence of said (desired) allelic variation with said optimized or validated detection assay; and
selecting an individuum comprising said (desired) allelic variation;
optionally propagating said individuum.

3. The method according to claim 1 or 2, wherein said assay is a hybridization-based assay or a PCR-based assay, preferably KASP or microarray.

4. The method according to any of claims 1 to 3, wherein optimizing said assay comprises comparing multiple primers or probes and/or the optimized assay comprises primers or probes which best discriminate between said desired allelic variation and said other allelic variation.

5. The method according to any of claims 1 to 4, wherein said synthetic oligonucleotide has a length of between 50 and 500 nucleotides, preferably between 80 and 200 nucleotides.

6. The method according to any of claims 1 to 5, wherein said synthetic oligonucleotide comprising said desired allelic variation is combined with one or more additional synthetic oligonucleotides comprising a different allelic variation.

7. The method according to any of claims 1 to 6, wherein said synthetic oligonucleotide is spiked in genomic DNA, preferably wild type genomic DNA from the same species as the population.

8. The method according to any of claims 1 to 7, wherein said population is mutagenized, preferably random mutagenesis such as ENU or EMS based mutagenesis.

9. The method according to any of claims 1 to 8, wherein said allelic variation is a a point mutation, a nonsense mutation, a knockout mutation, or a SNP.

10. The method according to any of claims 1 to 9, wherein said population is a population of plants or plant parts, preferably wherein said plant part is a plant organ or plant cell, such as a plant seed.

11. The method according to any of claims 1 to 10, wherein:
said population is a population of sugar beets
said synthetic oligonucleotide comprises the sequence of SEQ ID NO: 19 or 20, or a fragment thereof comprising the sequence of SEQ ID NO: 51 or SEQ ID NO: 52.

12. The method according to any of claims 1 to 10, wherein:
said population is a population of maize
said synthetic oligonucleotide comprises the sequence of SEQ ID NO: 42 or 43, or a fragment thereof comprising the sequence of SEQ ID NO: 53 or SEQ ID NO: 54.

13. A synthetic oligonucleotide suitable for use in the method according to any of claims 1 to 12, preferably wherein said oligonucleotide comprises the sequence of SEQ ID NO: 19 or 20, or a fragment thereof comprising the sequence of SEQ ID NO: 51 or SEQ ID NO: 52, or wherein said oligonucleotide comprises the sequence of SEQ ID NO: 42 or 43, or a fragment thereof comprising the sequence of SEQ ID NO: 53 or SEQ ID NO: 54.

14. Use of a synthetic oligonucleotide comprising a desired allelic variation for optimizing or validating an assay for detecting said allelic variation in a population.

15. A plant selected by the method according to claim 1 or generated by the method according to claim 2 or a part, seed or progeny of the plant, wherein the plant, the part, seed or progeny thereof exhibit the desired allelic variation.
